# EUROPEAN PATENT SPECIFICATION

(11) **EP 0 912 576 B1**
(45) Date of publication and mention of the grant of the patent: **12.12.2001**
(21) Application number: 97934060.1
(22) Date of filing: 08.07.1997
(51) Int. Cl.: C07D 477/08

(54) **PROCESS FOR SYNTHESIZING CARBAPENEM ANTIBIOTICS**
VERFAHREN ZUR HERSTELLUNG VON CARBAPENEM-ANTIBIOTIKA
PROCEDE DE SYNTHESE D'ANTIBIOTIQUES DE TYPE CARBAPENEME

(30) Priority: 12.07.1996 US 21649 P; 20.08.1996 GB 9617417
(43) Date of publication of application: 06.05.1999
(73) Proprietor: MERCK & CO., INC., Rahway, New Jersey 07065 (US)
(72) Inventor: WILLIAMS, John, M., Rahway, NJ 07065 (US); JOBSON, Ronald, B., Rahway, NJ 07065 (US)
(74) Representative: Hiscock, Ian James
(86) International application number: US9711844
(87) International publication number: WO9802439

(56) References cited:
- WO-A-93/15078
- M SUNAGAWA ET AL: "A novel carbapenem antibiotic, Sm-7338" THE JOURNAL OF ANTIBIOTICS, vol. 43, no. 5, 1990, pages 519-532, XP002043353
- M SUNAGAWA ET AL: "A new series of carbapenem antibiotics with 5'-substituted pyrrolidinylthio group at C-2 position" THE JOURNAL OF ANTIBIOTICS, vol. 44, no. 4, 1991, pages 459-462, XP002043354

## Description

### BACKGROUND OF THE INVENTION

The present invention relates to the synthesis of carbapenem antibiotics. It has been discovered that the inclusion of a secondary amine in the coupling reactions described herein significantly and surprisingly enhances the rate of reaction, allowing for shorter reaction times and relatively complete conversion at lower temperatures than observed when tertiary amines are used in this type of reaction.

Triethyl and diisopropylethyl amines have been reported to be useful in these reactions, but the reaction times and conditions have been unacceptable from a commercial standpoint. For example, WO 93/15078 published on August 5, 1993, relates to similar reactions in the presence of a tertiary amine, such as diisopropylethylamine, or an inorganic base, such as an alkali metal carbonate, e.g., potassium carbonate. Such reactions are performed at a temperature between -25°C and ambient temperature.

One object of the present invention is to effect a reaction between the carbapenem base molecule and the 2-position side chain which is sufficiently rapid and efficient to minimize the formation of side reaction products, and to avoid the need for inappropriate high and low temperatures and other reaction conditions.

Another object of the present invention is to avoid the use of catalysts and other reaction components which would require a separate removal step if traces thereof were contained in the final product.

These and other objects will be apparent from the teachings contained herein.

### SUMMARY OF THE INVENTION

A process for synthesizing a compound of the formula I: 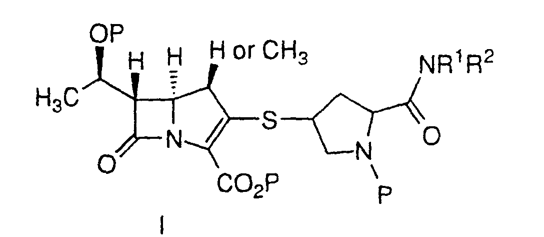 or a pharmaceutically acceptable salt or ester thereof, wherein each P independently represents H or a carboxyl, amino or hydroxyl protecting group, and R¹ and R² independently represent H, C₁₋₁₀alkyl (optionally substituted with a C₃₋₁₅cycloalkyl group or containing or interrupted by a C₃₋₁₅cycloalkylene portion), C₃₋₁₀cycloalkyl, aryl or heteroaryl, or C₁₋₁₀alkyl, aryl or heteroaryl substituted with from 1 to 3 groups selected from halo, hydroxy, cyano, -C(O)C₁₋₆alkyl, -C(O)-aryl, -NHC(O)C₁₋₆alkyl, -NHC(O)aryl, -OC₁₋₆alkylaryl, -SO₂C₁₋₆alkyl, arylsulfonyl, -NHSO₂C₁₋₆alkyl, -NHSO₂aryl, -C(O)NHC₁₋₆alkyl, C₁₋₁₅alkyl (optionally substituted with a C₃₋₁₅cycloalkyl group or containing or interrupted by a C₃₋₁₅cycloalkylene portion), C₃₋₁₅cycloalkyl, C₁₋₆alkoxy, aryl, aryloxy, -OC₁₋₆alkylaryl, amino, C₁₋₆alkylamino, di(C₁₋₆alkyl)amino, carboxy and sulfonylamino,
comprising reacting the compounds: 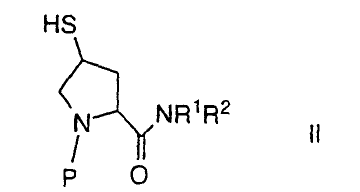 or a pharmaceutically acceptable salt or ester thereof, and 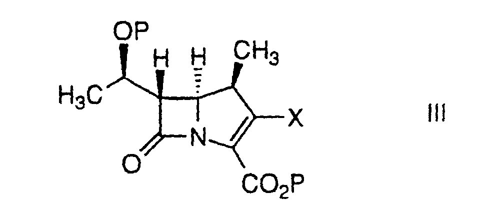 or a pharmaceutically acceptable salt or ester thereof, wherein X represents OP(O)(OR)₂, or OSO₂R, wherein R represents C₁₋₆ alkyl, (optionally substituted with a C₃₋₁₅cycloalkyl group or containing or interrupted by a C₃₋₁₅cycloalkylene portion), C₃₋₆cycloalkyl, C₁₋₆alkaryl, aryl or perfluoroC₁₋₆alkyl, in the presence of an amine selected from the group consisting of: diisopropylamine (DIPA), dicyclohexylamine (DCHA), 2,2,6,6-tetramethylpiperidine (TMP), 1,1,3,3-tetramethylguanidine (TMG), 1,8-diazabicyclo[4.3.0]undec-7-ene (DBU) and 1,5-diazabicyclo[4.3.0]non-5-ene (DBN) to produce a compound of formula I.

### DETAILED DESCRIPTION OF THE INVENTION

It has been discovered that the amines described herein facilitate a reaction between compounds of formulas II and III. With respect to compounds of formulas II and III, these compounds can be made in accordance with techniques such as those which are disclosed in U. S. Pat. Nos. 5,034,384 granted on July 23, 1991, and 4,994,568 granted on February 19, 1991, incorporated herein by reference.

Compounds such as compound IIa and IIIa, can likewise be produced in accordance with U. S. Pat. No. 5,478,820 granted on December 26, 1995, incorporated herein by reference.

The reactions described herein are preferably run in a polar aprotic solvent. Preferred examples of polar aprotic solvents include dimethylformamide, N-ethylpyrrolidinone and acetonitrile.

In a preferred aspect of the invention a process for synthesizing a compound of the formula Ia: 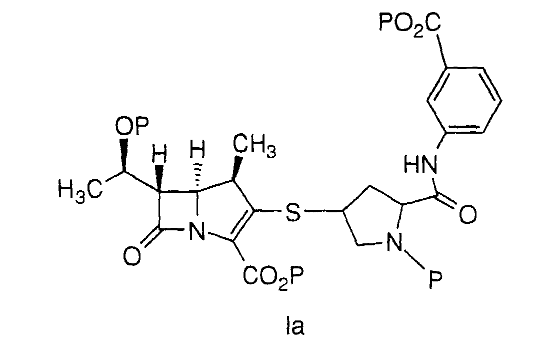 or a pharmaceutically acceptable salt or ester thereof, is described herein wherein each P independently represents H or a protecting group, comprising:
reacting the compounds: 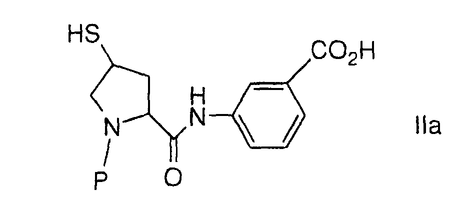 or a pharmaceutically acceptable salt or ester thereof, and 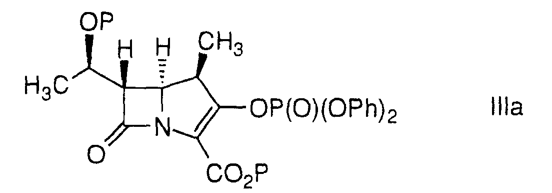 or a pharmaceutically acceptable salt or ester thereof, wherein Ph represents phenyl, in the presence of an amine selected from the group consisting of:
diisopropylamine (DIPA), dicyclohexylamine (DCHA), 2,2,6,6-tetramethylpiperidine (TMP), 1,1,3,3-tetramethylguanidine (TMG), 1,8-diazabicyclo[4.3.0]undec-7-ene (DBU) and 1,5-diazabicyclo[4.3.0]non-5-ene (DBN)
to produce a compound of formula Ia.

The invention is described herein in detail using the terms defined below unless otherwise specified.

The term "alkyl" refers to a monovalent alkane (hydrocarbon) derived radical containing from 1 to 15 carbon atoms unless otherwise defined. It may be straight or branched, and when of sufficient size, e.g., C₃₋₁₅ may be cyclic. Preferred straight or branched alkyl groups include methyl, ethyl, propyl, isopropyl, butyl and t-butyl. Preferred cycloalkyl groups include cyclopropyl, cyclopentyl and cyclohexyl.

Alkyl also includes an alkyl group substituted with a cycloalkyl group, such as cyclopropylmethyl.

Alkyl also includes a straight or branched alkyl group which contains or is interrupted by a cycloalkylene portion.
Examples include the following: 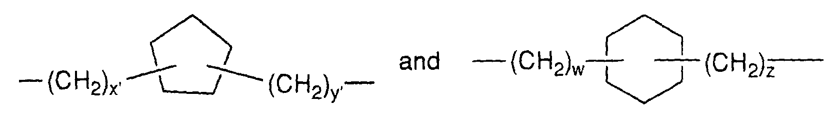 wherein: x' and y' = from 0-10; and w and z = from 0-9.

The alkylene and monovalent alkyl portion(s) of the alkyl group can be attached at any available point of attachment to the cycloalkylene portion.

When substituted alkyl is present, this refers to a straight, branched or cyclic alkyl group as defined above, substituted with 1-3 groups as defined with respect to each variable.

Heteroalkyl means an alkyl group containing from 2-15 carbon atoms and being interrupted by from 1-4 heteroatoms selected from O, S and N.

The term "alkenyl" refers to a hydrocarbon radical straight, branched or cyclic containing from 2 to 15 carbon atoms and at least one carbon to carbon double bond. Preferably one carbon to carbon double bond is present, and up to four nonaromatic (non-resonating) carbon-carbon double bonds may be present. Preferred alkenyl groups include ethenyl, propenyl, butenyl and cyclohexenyl. As described above with respect to alkyl, the straight, branched or cyclic portion of the alkenyl group may contain double bonds and may be substituted when a substituted alkenyl group is provided.

The term "alkynyl" refers to a hydrocarbon radical straight, branched or cyclic, containing from 2 to 15 carbon atoms and at least one carbon to carbon triple bond. Up to three carbon-carbon triple bonds may be present. Preferred alkynyl groups include ethynyl, propynyl and butynyl. As described above with respect to alkyl, the straight, branched or cyclic portion of the alkynyl group may contain triple bonds and may be substituted when a substituted alkynyl group is provided.

Aryl refers to aromatic rings e.g., phenyl, substituted phenyl and like groups as well as rings which are fused, e.g., naphthyl and the like. Aryl thus contains at least one ring having at least 6 atoms, with up to two such rings being present, containing up to 10 atoms therein, with alternating (resonating) double bonds between adjacent carbon atoms. The preferred aryl groups are phenyl and naphthyl. Aryl groups may likewise be substituted as defined below. Preferred substituted aryls include phenyl and naphthyl substituted with one to three groups, such as selected from halo, alkyl and trifluoromethyl.

The term "heteroaryl" refers to a monocyclic aromatic hydrocarbon group having 5 or 6 ring atoms, or a bicyclic aromatic group having 8 to 10 atoms, containing at least one heteroatom, O, S or N, in which a carbon or nitrogen atom is the point of attachment, and in which one additional carbon atom is optionally replaced by a heteroatom selected from O or S, and in which from 1 to 3 additional carbon atoms are optionally replaced by nitrogen heteroatoms. The heteroaryl group is optionally substituted with up to three groups.

Heteroaryl includes aromatic and partially aromatic groups which contain one or more heteroatoms. Examples of this type are thiophene, purine, imidazopyridine, pyridine, oxazole, thiazole, oxazine, pyrazole, tetrazole, imidazole, pyridine, pyrimidine, pyrazine and triazine. Examples of partially aromatic groups are tetrahydroimidazo[4,5-c]pyridine, phthalidyl and saccharinyl, as defined below.

Substituted alkyl, aryl and heteroaryl, and the substituted portions of aralkyl, aralkoxy, heteroaralkyl, heteroaralkoxy and like groups are substituted with from 1-3 groups selected from the group consisting of: halo, hydroxy, cyano, acyl, acylamino, aralkoxy, alkylsulfonyl, arylsulfonyl, alkylsulfonylamino, arylsulfonylamino, alkylaminocarbonyl, alkyl, alkoxy, aryl, aryloxy, aralkoxy, amino, alkylamino, dialkylamino, carboxy and sulfonylamino.

The terms "heterocycloalkyl" and "heterocyclyl" refer to a cycloalkyl group (nonaromatic) in which one of the carbon atoms in the ring is replaced by a heteroatom selected from O, S(O)_{y} or N, and in which up to three additional carbon atoms may be replaced by said heteroatoms. When three heteroatoms are present in the heterocycle, they are not all linked together.

Examples of heterocyclyls are piperidinyl, morpholinyl, azetidinyl, pyrrolidinyl, tetrahydrofuranyl, imidazolinyl, piperazinyl, pyrolidin-2-one, piperidin-2-one and the like.

Acyl as used herein refers to -C(O)C₁₋₆ alkyl and -C(O)-aryl.

Acylamino refers to the group -NHC(O)C₁₋₆ alkyl and -NHC(O)aryl.

Aralkoxy refers to the group -OC₁₋₆ alkylaryl.

Alkaryl refers to C₁₋₆ alkyl-aryl-.

Alkylsulfonyl refers to the group -SO₂C₁₋₆ alkyl.

Alkylsulfonylamino refers to the group -NHSO₂C₁₋₆alkyl.

Arylsulfonylamino refers to the group -NHSO₂aryl.

Alkylaminocarbonyl refers to the group -C(O)NHC₁₋₆ alkyl.

Aryloxy refers to the group -O-aryl.

Sulfonylamino refers to the group -NHSO₃H.

Halo means Cl, F, Br and I selected on an independent basis.

The compounds of the present invention are useful in various pharmaceutically acceptable salt forms. The term "pharmaceutically acceptable salt" refers to those salt forms which would be apparent to the pharmaceutical chemist. i.e., those which are substantially non-toxic and which provide the desired pharmacokinetic properties, palatability, absorption, distribution, metabolism or excretion. Other factors, more practical in nature, which are also important in the selection, are cost of the raw materials, ease of crystallization, yield, stability, hygroscopicity and flowability of the resulting bulk drug. Conveniently pharmaceutical compositions may be prepared from the active ingredients in combination with pharmaceutically acceptable carriers.

The pharmaceutically acceptable salts of the compounds of formula I include conventional non-toxic salts and quartemary ammonium salts of the compounds of formula I formed, e.g., with inorganic or organic cationic groups. For example, these salts include charge balancing cations which are present in association with the compound as necessary to maintain overall charge neutrality. Typically the charged specie would be a pharmaceutically acceptable salt-forming ion, such as sodium, potassium, magnesium and the like. When the counterion includes a bis cationic specie, e.g., Ca⁺² an appropriate amount is typically present relative to the carbapenem moiety to provide overall charge neutrality. Thus, a half molar equivalent of Ca⁺² can be included to maintain overall charge neutrality. All such embodiments are included in the present invention.

Numerous salt-forming ions are recited in Berge, S. M., et al. **J. Pharm. Sci.** 66(1): 1-16 (1977), the teachings of which are incorporated herein by reference.

A preferred group of salt-forming cations is selected from the group consisting of: sodium, potassium, calcium and magnesium.

More preferably the cation represents a member selected from the group consisting of: Na⁺, Ca⁺² and K⁺.

The pharmaceutically acceptable salts of the present invention can be synthesized by conventional chemical methods. Generally, the salts are prepared by reacting the free base or acid with stoichiometric amounts or with an excess of the desired salt-forming inorganic or organic acid or base, in a suitable solvent or solvent combination.

One preferred group of amines for use in the present invention includes diisopropylamine and dicyclohexylamine. These amines form crystalline salts with diphenylphosphoric acid, which crystallize from the coupling reaction mixture, affording an opportunity for removal of the phosphoric acid by-product of the reaction.

Using the amines noted above, the reaction rate is increased unexpectedly.

The compounds formed in the present invention have asymmetric centers and occur as racemates, racemic mixtures, and as individual diastereomers. The processes of synthesizing all such isomers, including optical isomers, are included in the present invention.

The carboxyl group at the 3-position and the hydroxyl group at the 8-position of the carbapenem, the pyrrolidinyl nitrogen and when present, the m-carboxyphenyl moiety may remain blocked until the final product is prepared. These blocking groups are readily removable, i.e., they can be removed, if desired, by procedures which will not cause cleavage or other disruption of the remaining portions of the molecule. Such procedures include chemical and enzymatic hydrolysis, treatment with chemical reducing or oxidizing agents under mild conditions, treatment with fluoride ion, treatment with a transition metal catalyst and a nucleophile, and catalytic hydrogenation.

Examples of suitable hydroxyl protecting groups are: t-butylmethoxyphenylsilyl, t-butoxydiphenylsilyl, trimethylsilyl, triethylsilyl, o-nitrobenzyloxycarbonyl, p-nitrobenzyloxycarbonyl, benzyloxycarbonyl, t-butyloxycarbonyl, 2,2,2-trichloroethyloxycarbonyl and allyloxycarbonyl. Preferred hydroxyl protecting groups are trimethylsilyl and triethylsilyl.

Examples of suitable carboxyl protecting groups are: benzhydryl, o-nitrobenzyl, p-nitrobenzyl, 2-naphthylmethyl, allyl, 2-chloroallyl, benzyl, 2,2,2-trichloroethyl, trimethylsilyl, t-butyldimethylsilyl, t-butyldiphenylsilyl, 2-(trimethylsilyl)ethyl, phenacyl, p-methoxybenzyl, acetonyl, p-methoxyphenyl, 4-pyridylmethyl and t-butyl. A preferred carboxyl protecting group is p-nitrobenzyl.

Many other suitable hydroxyl and carboxyl protecting groups are known in the art. See, e.g., T.W. Greene, Protective Groups in Organic Synthesis, John Wiley & Sons, Inc., 1981 (Chapters 2 and 5).

The invention is illustrated in connection with the following non-limiting examples.

### PREPARATIVE EXAMPLE 1

### (2S,4S)-1-(p-Nitrobenzyloxycarbonyl)-2-(3-carboxyphenylcarbamoyl)-pyrrolidin-4-ylthiol: Sidechain thiol (B)

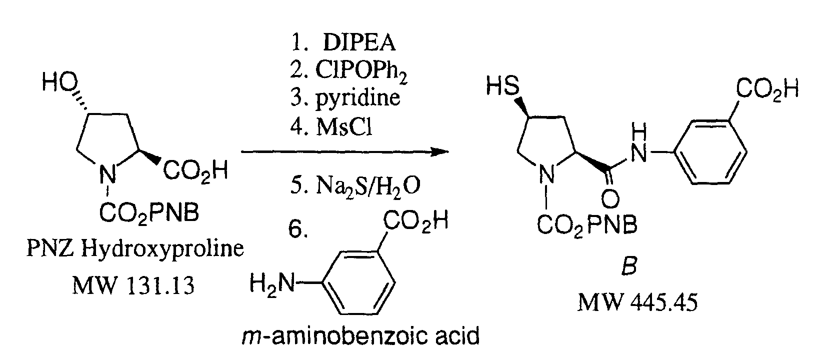

PNZ hydroxyproline (15 g) was stirred in a mixture of THF (225 mL) and toluene(75 mL), and the mixture was cooled to -10°C. Diisopropylethylamine (13.4 g) was added followed by a solution of 12.1 g of diphenylphosphinic chloride in 15 mL of toluene. The mixture was aged for 2 h at -10°C and 4.1 g of pyridine in 4 mL of toluene was added followed by 6.29 g of methanesulfonyl chloride in 4 mL of toluene. After 4h a solution of sodium sulfide trihydrate (7.0 g) in 75 mL of water was added and the mixture was warmed to 20°C and aged for 14 h. The layers were separated and the organic layer was extracted with HCl (150 mL, 1.0 N), sodium bicarbonate (280 mL, 5%, 40-45°C), and aqueous NaCl (150 mL, saturated, 20-25°C).

To a 1 L flask was charged 540 mL of the extract, 6.6 g of *m*-aminobenzoic acid, and 1.0 mL of tributylphosphine. The mixture was degassed and was concentrated by atmospheric distillation to 200 mL. Toluene was added with continued distillation until the distillate temperature reached 110 °C. Acetic acid (200 mL) and 1-propanol (200 mL) were added and the resulting mixture was cooled to 15-20 °C, and aged for 18 h. The product was isolated by filtration washing with 1-propanol and dried under vacuum at 80°C to give 15.4 g of sidechain thiol.

### EXAMPLE 1

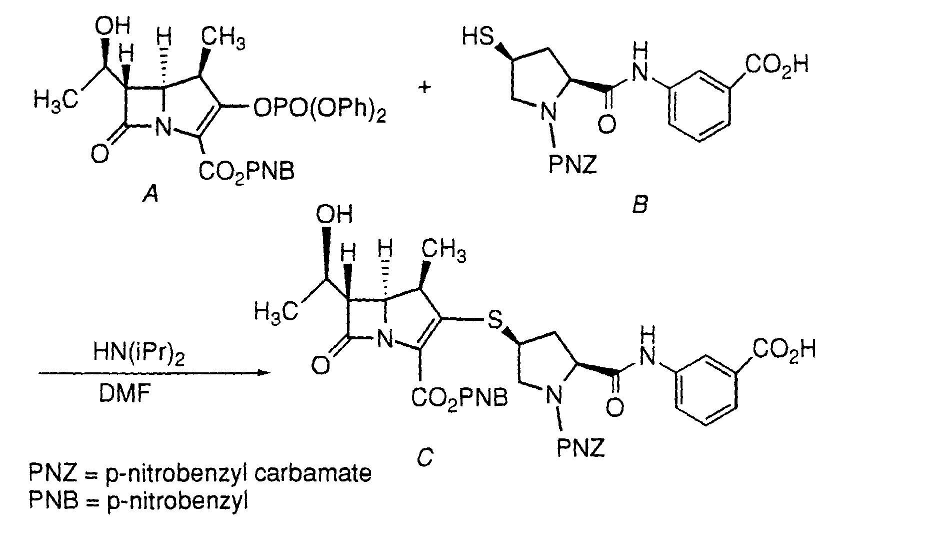

Compound *A* (594 mg, 1.0 mmol) and compound *B* (454 mg, 1.02 mmol) were combined and DMF (2.0 mL) was added. The mixture was degassed. The resulting solution was cooled to -50 to -30°C. Diisopropylamine (204 mg, 0.28 mL) was added. The reaction was complete to >98% conversion in 2-3 hrs.

### COMPARATIVE EXAMPLE

The procedure of Example One was followed, except that diisopropylamine was replaced with an equal molar quantity of diisopropylethylamine. The reaction was complete (98% conversion) after 18 h.

### EXAMPLE 2

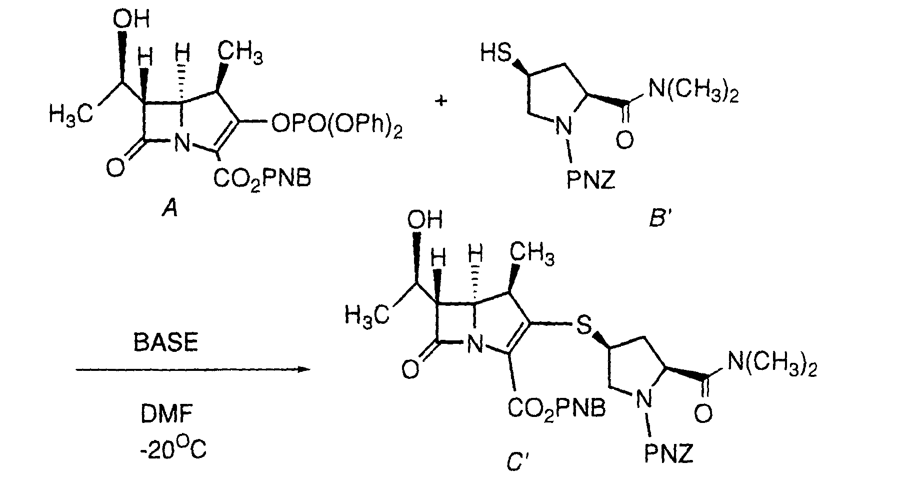

Using the procedure set forth in Example One, the base diisopropylamine was replaced with diisopropylethylamine (DIPEA).

The rate of reaction between A and B' to produce C' is significantly higher for DIPA and the other amines recited than for DIPEA.

**TABLE I**

| | *time to 98% conversion* | *Assay Yield (%)* | *Area % purity* |
|---|---|---|---|
| DBU | <0.25 h | - | - |
| DIPA | 0.5 h | 98 | 97.3 |
| TMG | 2h | 98 | 98.0 |
| TMP | 2h | - | - |
| DIPEA | >4 h^{a} | 92^{b} | - |

| | | | |
|---|---|---|---|
| Reactions were conducted using equimolar amounts of A and B' at 0.1 M with 1.1 equiv of base at -20 °C. ^{a} about 90% conversion at 4 h. ^{b} yield at 4 h. | | | |

## Claims

1. A process for synthesizing a compound of the formula I: 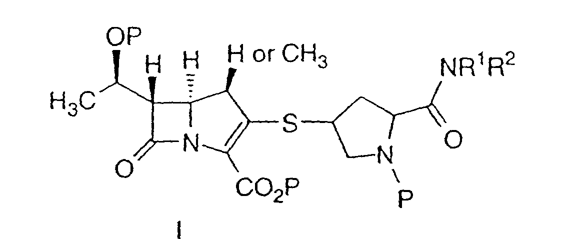 or a pharmaceutically acceptable salt or ester thereof, wherein each P independently represents H or a carboxyl, amino or hydroxyl protecting group, and R¹ and R² independently represent H, C₁₋₁₀alkyl (optionally substituted with a C₃₋₁₅cycloalkyl group or containing or interrupted by a C₃₋₁₅cycloalkylene portion), C₃₋₁₀cycloalkyl, aryl or heteroaryl, or C₁₋₁₀alkyl, aryl or heteroaryl substituted with from 1 to 3 groups selected from halo, hydroxy, cyano, -C(O)C₁₋₆alkyl, -C(O)-aryl, -NHC(O)C₁₋₆alkyl, -NHC(O)aryl, -OC₁₋₆alkylaryl, -SO₂C₁₋₆alkyl, arylsulfonyl, -NHSO₂C₁₋₆alkyl, -NHSO₂aryl, -C(O)NHC₁₋₆alkyl, C₁₋₁₅alkyl (optionally substituted with a C₃₋₁₅cycloalkyl group or containing or interrupted by a C₃₋₁₅cycloalkylene portion), C₃₋₁₅cycloalkyl, C₁₋₆alkoxy, aryl, aryloxy, -OC₁₋₆alkylaryl, amino, C₁₋₆alkylamino, di(C₁₋₆alkyl)amino, carboxy and sulfonylamino,
wherein said heteroaryl is an aromatic or partially aromatic monocyclic hydrocarbon having 5 to 6 ring atoms or bicyclic hydrocarbon having 8 to 10 ring atoms, containing one heteroatom selected from O, S or N, in which a carbon or nitrogen atom is the point of attachment, and in which at least one additional carbon atom is optionally replaced by a heteroatom selected from O or S, and in which at least 1 to 3 additional carbon atoms are optionally replaced by nitrogen heteroatoms;
comprising reacting the compounds: 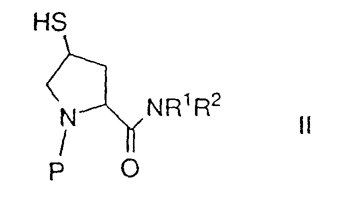 or a pharmaceutically acceptable salt or ester thereof, and 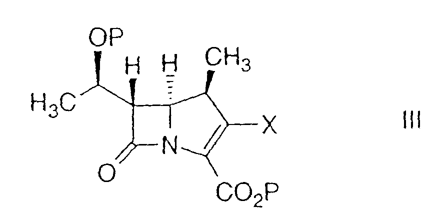 or a pharmaceutically acceptable salt or ester thereof, wherein X represents OP(O)(OR)₂, or OSO₂R, wherein R represents C₁₋₆ alkyl, (optionally substituted with a C₃₋₁₅cycloalkyl group or containing or interrupted by a C₃₋₁₅cycloalkylene portion), C₃₋₆cycloalkyl, C₁₋₆alkaryl, aryl or perfluoroC₁₋₆alkyl, in the presence of an amine selected from the group consisting of:
diisopropylamine (DIPA), dicyclohexylamine (DCHA), 2,2,6,6-tetramethylpiperidine (TMP), 1,1,3,3-tetramethylguanidine (TMG), 1,8-diazabicyclo[4.3.0]undec-7-ene (DBU) and 1,5-diazabicyclo[4.3.0]non-5-ene (DBN)
to produce a compound of formula I.

2. A process for synthesizing a compound of the formula Ia: 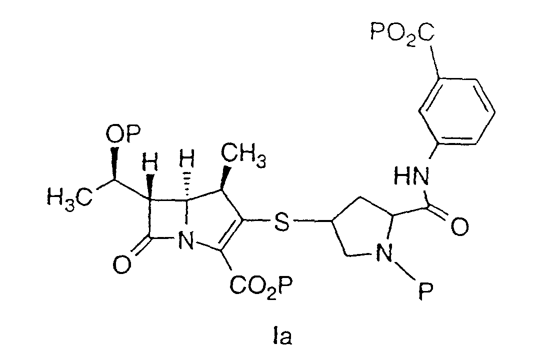 or a pharmaceutically acceptable salt or ester thereof, wherein each P independently represents H or a carboxyl, amino or hydroxyl protecting group, comprising:
reacting the compounds: 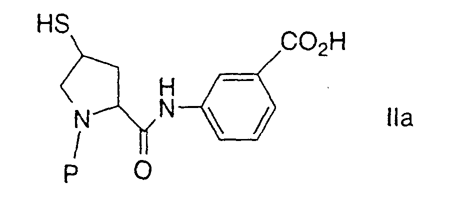 or a pharmaceutically acceptable salt or ester thereof, and 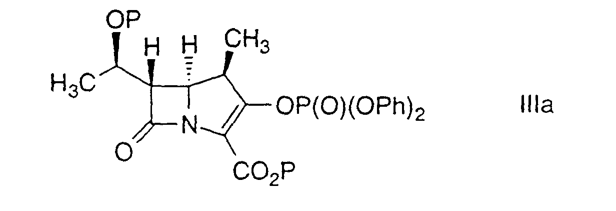 or a pharmaceutically acceptable salt or ester thereof, wherein Ph represents phenyl, in the presence of an amine selected from the group consisting of:
diisopropylamine (DIPA), dicyclohexylamine (DCHA), 2,2,6,6-tetramethylpiperidine (TMP), 1,1,3,3-tetramethylguanidine (TMG), 1,8-diazabicyclo[4.3.0]undec-7-ene (DBU) and 1,5-diazabicyclo[4.3.0]non-5-ene (DBN)
to produce a compound of formula Ia.

3. A process in accordance with claim 1 wherein the secondary amine is selected from the group consisting of:
diisopropylamine (DIPA) and dicyclohexylamine (DCHA).

4. A process in accordance with claim 3 wherein the synthesis is run in a polar aprotic solvent.

5. A process in accordance with claim 4 wherein the polar aprotic solvent is selected from the group consisting of dimethylformamide, N-ethylpyrrolidinone and acetonitrile.

## Patentansprüche

1. Ein Verfahren zur Synthese einer Verbindung der Formel I: 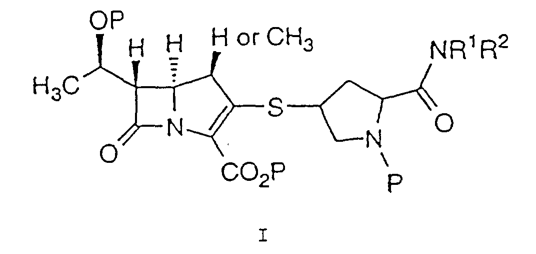 oder eines pharmazeutisch annehmbaren Salzes oder Esters davon, wobei jeder Rest P unabhängig H oder eine Carboxyl-, Amino- oder Hydroxyl-Schutzgruppe bedeutet und R¹ und R² unabhängig H, C₁₋₁₀-Alkyl (das gegebenenfalls mit einer C₃₋₁₅-Cycloalkylgruppe substituiert ist oder einen C₃₋₁₅-Cycloalkylen-Teil enthält oder von einem C₃₋₁₅-Cycloalkylen-Teil unterbrochen ist), C₃₋₁₀-Cycloalkyl, Aryl oder Heteroaryl, oder C₁₋₁₀-Alkyl, Aryl oder Heteroaryl, substituiert mit 1 bis 3 Gruppen, ausgewählt aus Halogen, Hydroxy, Cyano, -C(O)C₁₋₆-Alkyl, -C(O)-Aryl, -NHC(O)-C₁₋₆-Alkyl, -NHC(O)-Aryl, -OC₁₋₆-Alkylaryl, -SO₂C₁₋₆-Alkyl, Arylsulfonyl, -NHSO₂C₁₋₆-Akyl, -NHSO₂-Aryl, -C(O)NHC₁₋₆-Alkyl, C₁₋₁₅-Alkyl (das gegebenenfalls mit einer C₃₋₁₅-Cycloalkylgruppe substituiert ist oder einen C₃₋₁₅-Cycloalkylen-Teil enthält oder von einem C₃₋₁₅-Cycloalkylen-Teil unterbrochen ist) , C₃₋₁₅-Cycloalkyl, C₁₋₆-Alkoxy, Aryl, Aryloxy, -OC₁₋₆-Alkylaryl, Amino, C₁₋₆-Alkylamino, Di(C₁₋₆-Alkyl)amino, Carboxy und Sulfonylamino, sind, wobei das Heteroaryl ein aromatischer oder teilweise aromatischer monocyclischer Kohlenwasserstoff mit 5 bis 6 Ringatomen oder ein bicyclischer Kohlenwasserstoff mit 8 bis 10 Ringatomen ist, der ein Heteroatom enthält, ausgewählt aus O, S oder N, worin ein Kohlenstoff- oder Stickstoffatom der Verknüpfungspunkt ist und worin wenigstens ein zusätzliches Kohlenstoffatom gegebenenfalls durch ein Heteroatom, ausgewählt aus O oder S, ersetzt ist und worin wenigstens 1 bis 3 zusätzliche Kohlenstoffatome gegebenenfalls durch Stickstoff-Heteroatome ersetzt sind, umfassend die Umsetzung der Verbindungen: 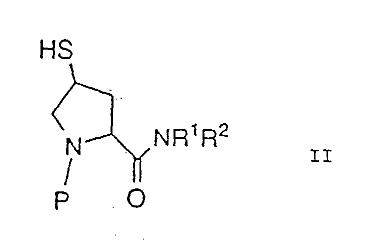 oder eines pharmazeutisch annehmbaren Salzes oder Esters davon und 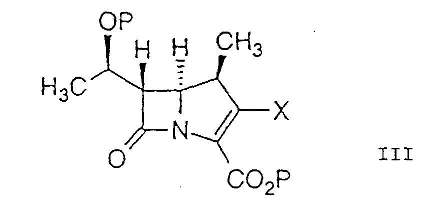 oder eines pharmazeutisch annehmbaren Salzes oder Esters davon, worin X OP(O)(OR)₂ oder OSO₂R ist, worin R C₁₋₆-Alkyl (das gegebenenfalls mit einer C₃₋₁₅-Cycloalkylgruppe substituiert ist oder einen C₃₋₁₅-Cycloalkylen-Teil enthält oder von einem C₃₋₁₅-Cycloalkylen-Teil unterbrochen ist), C₃₋₆-Cycloalkyl, C₁₋₆-Alkaryl, Aryl oder Perfluor-C₁₋₆-alkyl ist, in Gegenwart eines Amins, ausgewählt aus der Gruppe, bestehend aus:
Diisopropylamin (DIPA), Dicyclohexylamin (DCHA), 2,2,6,6-Tetramethylpiperidin (TMP), 1,1,3,3-Tetramethylguanidin (TMG), 1,8-Diazabicyclo[4.3.0]-undec-7-en (DBU) und 1,5-Diazabicyclo[4.3.0]non-5-en (DBN),
um eine Verbindung der Formel I zu erzeugen.

2. Ein Verfahren zur Synthese einer Verbindung der Formel Ia: 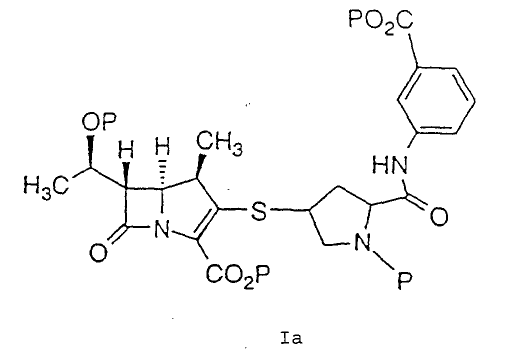 oder eines pharmazeutisch annehmbaren Salzes oder Esters davon, wobei jeder Rest P unabhängig H oder eine Carboxyl-, Amino- oder Hydroxyl-Schutzgruppe bedeutet, umfassend:
die Umsetzung der Verbindungen: 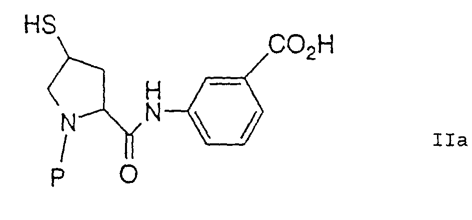 oder eines pharmazeutisch annehmbaren Salzes oder Esters davon und 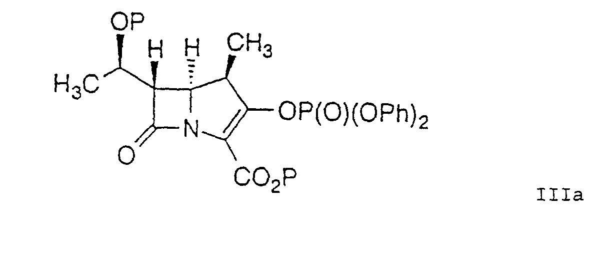 oder eines pharmazeutisch annehmbaren Salzes oder Esters davon, worin Ph Phenyl bedeutet, in Gegenwart eines Amins, ausgewählt aus der Gruppe, bestehend aus:
Diisopropylamin (DIPA), Dicyclohexylamin (DCHA), 2,2,6,6-Tetramethylpiperidin (TMP), 1,1,3,3-Tetramethylguanidin (TMG), 1,8-Diazabicyclo[4.3.0]-undec-7-en (DBU) und 1,5-Diazabicyclo[4.3.0]non-5-en (DBN),
um eine Verbindung der Formel Ia zu erzeugen.

3. Ein Verfahren gemäß Anspruch 1, bei dem das sekundäre Amin ausgewählt ist aus der Gruppe, bestehend aus: Diisopropylamin (DIPA) und Dicyclohexylamin (DCHA).

4. Ein Verfahren gemäß Anspruch 3, bei dem die Synthese in einem polaren aprotischen Lösungsmittel durchgeführt wird.

5. Ein Verfahren gemäß Anspruch 4, bei dem das polare aprotische Lösungsmittel ausgewählt ist aus der Gruppe, bestehend aus Dimethylformamid, N-Ethylpyrrolidinon und Acetonitril.

## Revendications

1. Procédé pour la synthèse d'un composé de formule I, 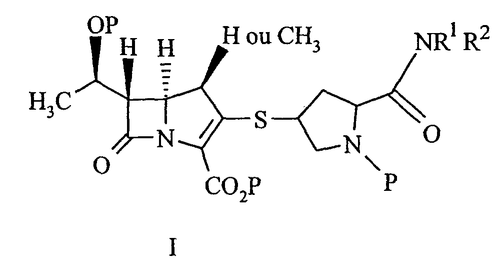 ou d'un de ses sels ou esters pharmaceutiquement acceptables, dans lequel chaque P représente de façon indépendante H ou un groupe protecteur de carboxyle, d'amino ou d'hydroxyle, et R¹ et R² représentent, de façon indépendante, H, un alkyle en C₁₋₁₀ (éventuellement substitué par un groupe cycloalkyle en C₃₋₁₅ ou contenant, ou interrompu par, un fragment cyclo-alkylène en C₃₋₁₅), un cycloalkyle en C₃₋₁₀, un aryle ou hétéroaryle, ou un alkyle en C₁₋₁₀, un aryle ou hétéroaryle substitué par 1 à 3 groupes choisis parmi les groupes halogéno, hydroxyle, cyano, -C(O)(alkyle en C₁₋₆),-C(O)aryle, -NHC(O)(alkyle en C₁₋₆), -NHC(O)aryle, -O(alkyle en C₁₋₆)aryle, -SO₂-(alkyle en C₁₋₆), arylsulfonyle, -NHSO₂(alkyle en C₁₋₆), -NHSO₂aryle,-C(O)-NH(alkyle en C₁₋₆), alkyle en C₁₋₁₅ (éventuellement substitué par un groupe cycloalkyle en C₃₋₁₅ ou contenant, ou interrompu par, un fragment cyclo-alkylène en C₃₋₁₅), cycloalkyle en C₃₋₁₅, alcoxy en C₁₋₆, aryle, aryloxy,-O-(alkyl en C₁₋₆)aryle, amino, (alkyl en C₁₋₆)amino, di(alkyl en C₁₋₆)amino, carboxyle et sulfonylamino,
où ledit hétéroaryle est un hydrocarbure monocyclique, aromatique ou partielle-ment aromatique, dont le squelette cyclique comprend 5 à 6 atomes, ou un hydrocarbure bicyclique dont le squelette cyclique comprend 8 à 10 atomes, contenant un hétéroatome choisi parmi O, S ou N, le point d'attachement étant un atome de carbone ou d'azote, et dans lequel au moins un atome de carbone supplémentaire est éventuellement remplacé par un hétéroatome choisi parmi O ou S et dans lequel au moins 1 à 3 atomes de carbone supplémentaires sont éventuellement remplacés par des hétéroatomes d'azote ;
comprenant la réaction des composés : 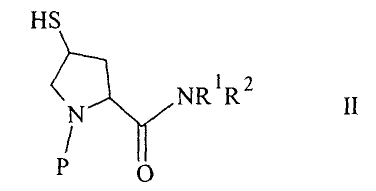 ou un de ses sels ou esters pharmaceutiquement acceptables et 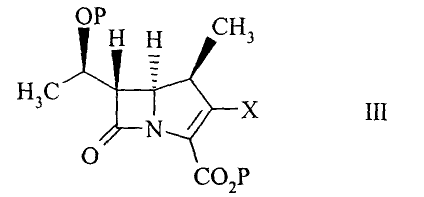 ou un de ses sels ou esters pharmaceutiquement acceptables, où X représente OP(O)(OR)₂, ou OSO₂R, où R représente un alkyle en C₁₋₆ (éventuellement substitué par un groupe cycloalkyle en C₃₋₁₅ ou contenant, ou interrompu par, un fragment cycloalkylène en C₃₋₁₅), un cycloalkyle en C₃₋₆, un alkylaryle en C₁₋₆, un aryle ou un perfluoro(alkyle en C₁₋₆), en présence d'une amine choisie dans le groupe constitué par la diisopropylamine (DIPA), la dicyclohexylamine (DCHA), la 2,2,6,6-tétraméthylpipéridine (TMP), la 1,1,3,3-tétraméthyl-guanidine (TMG), le 1,8-diazabicyclo[4.3.0]undéc-7-ène (DBU) et le 1,5-diazabicyclo[4.3.0]non-5-ène (DBN)
pour produire un composé de formule I.

2. Procédé pour la synthèse d'un composé de formule la, 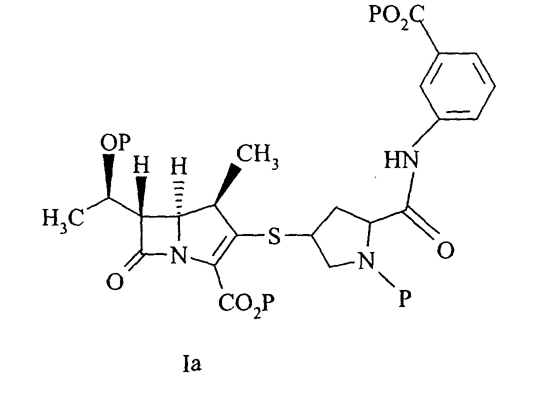 ou d'un de ses sels ou esters pharmaceutiquement acceptables, dans lequel chaque P représente de façon indépendante H ou un groupe protecteur de carboxyle, d'amino ou d'hydroxyle, comprenant :
la réaction des composés : 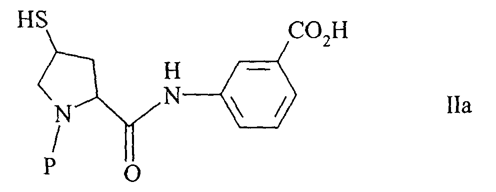 ou un de ses sels ou esters pharmaceutiquement acceptables et 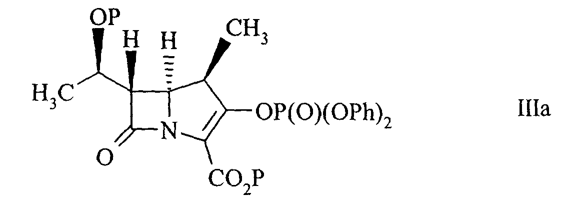 ou un de ses sels ou esters pharmaceutiquement acceptables, où Ph représente un phényle, en présence d'une amine choisie dans le groupe constitué par la diisopropylamine (DIPA), la dicyclohexylamine (DCHA), la 2,2,6,6-tétra-méthylpipéridine (TMP), la 1,1,3,3-tétraméthylguanidine (TMG), le 1,8-diaza-bicyclo[4.3.0]undéc-7-ène (DBU) et le 1,5-diazabicyclo[4.3.0]non-5-ène (DBN) pour produire un composé de formule la.

3. Procédé selon la revendication 1 où l'amine secondaire est choisie dans le groupe constitué par la diisopropylamine (DIPA) et la dicyclohexylamine (DCHA).

4. Procédé selon la revendication 3, dans lequel la synthèse est effectuée dans un solvant aprotique polaire.

5. Procédé selon la revendication 4, dans lequel le solvant aprotique polaire est choisi dans le groupe constitué par le diméthylformamide, la N-éthylpyrrolidinone et l'acétonitrile.
